(19) Europäisches Patentamt
European Patent Office
Office européen des brevets

(11) **EP 4 756 822 A1**

(12) **EUROPEAN PATENT APPLICATION**
published in accordance with Art. 153(4) EPC

(43) Date of publication:
**10.06.2026 Bulletin 2026/24**

(21) Application number: **23948610.3**

(22) Date of filing: **08.12.2023**

(51) International Patent Classification (IPC):
*G16H 20/13* (2018.01)    *G06V 10/10* (2022.01)
*G06V 30/41* (2022.01)    *G06V 10/75* (2022.01)
*G06V 10/74* (2022.01)

(52) Cooperative Patent Classification (CPC):
**G06V 10/10; G06V 10/74; G06V 10/75;
G06V 30/41; G16H 20/13**

(86) International application number:
**PCT/KR2023/020202**

(87) International publication number:
**WO 2025/033617 (13.02.2025 Gazette 2025/07)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **04.08.2023 KR 20230102468**

(71) Applicant: **Medility Inc.**
**Seoul 04213 (KR)**

(72) Inventors:
• **KIM, Ho Heon**
  **Seoul 04213 (KR)**
• **PARK, Sangeon**
  **Seoul 04213 (KR)**

(74) Representative: **Studio Torta S.p.A.**
**Via Viotti, 9**
**10121 Torino (IT)**

(54) **APPARATUS AND METHOD FOR INSPECTING MEDICINE PACKAGE**

(57) The present disclosure relates to a method for inspecting a medicine package performed by at least one processor. The method includes receiving a first image of a medicine package captured by a first camera, receiving a second image of the medicine package captured by a second camera, receiving medicine information related to the medicine package, and inspecting the medicine package based on the first image, the second image, and the medicine information, wherein the medicine package includes a plurality of tablets.

FIG. 1

**Description**

**BACKGROUND**

Field

The present disclosure relates to a medicine package inspection device and a method for inspection of a medicine package, specifically a method and system for inspecting a medicine package based on information obtained by captured images of the medicine package from various angles.

Description of Related Art

In pharmaceutical companies, pharmacies, hospitals, etc. involved in dispensing of pharmaceutical products, fully automated tablet dispensing and packaging system are used to automate dispensing operations. Medical personnel use the system to automatically sort and package medications according to prescriptions and to deliver the medications to patients. To prevent medication errors, a medication verification process is required before delivery to patients.

Medical accidents caused by medication errors may be life-threatening and highly sensitive issues. However, related companies, pharmacies, hospitals, etc., face significant difficulties due to the time and labor wasted in manual and direct verification processes. In addition, a problem lies in that an automated medication verification process has low accuracy.

**SUMMARY**

Devices, systems, etc. for automating medicine package inspection are being developed. Conventional medicine package inspection devices have the problem of difficulty in identifying tablets within the package when the tablets are overlapping or upside down. Devices that perform object recognition based on a single captured image for medicine package inspection have low object recognition accuracy for the tablets inside the package.

The present disclosure provides a medicine package inspection device and a method for performing medicine package inspection based on images captured from multiple angles of the medicine package to resolve the issues described above.

The present disclosure may be implemented in various configurations including a method, a computer program stored in an apparatus (system) and/or a computer-readable storage medium, and the computer-readable storage medium that stores the computer program.

According to embodiments of the present disclosure, there is provided a method for inspecting a medicine package performed by at least one processor, the method including receiving a first image of a medicine package captured by a first camera, receiving a second image of the medicine package captured by a second camera, receiving medicine information related to the medicine package, and inspecting the medicine package based on the first image, the second image, and the medicine information, wherein the medicine package includes a plurality of tablets.

According to embodiments of the present disclosure, the inspecting of the medicine package may include performing object recognition on the first image by using an object recognition model and generating a first recognition result, performing object recognition on the second image by using the object recognition model and generating a second recognition result, calibrating the second recognition result by using a calibration parameter related to the first camera and the second camera, and inspecting the medicine package based on the first recognition result, the calibrated second recognition result, and the medicine information.

The inspecting of the medicine package may include performing object recognition on the first image by using an object recognition model and generating a first recognition result, transforming the second image into a third image by using a calibration parameter related to the first camera and the second camera, performing object recognition on the third image by using the object recognition model and generating a second recognition result, and inspecting the medicine package based on the first recognition result, the second recognition result, and the medicine information.

The first image may be obtained by capturing a first side of the medicine package, wherein the second image is obtained by capturing a second side of the medicine package.

The inspecting of the medicine package based on the first recognition result, the calibrated second recognition result, and the medicine information may include generating a third recognition result by merging the first recognition result and the calibrated second recognition result, and inspecting the medicine package by comparing at least part of the third recognition result with at least part of the medicine information.

The medicine information may include prescription data, wherein the generating of the first recognition result includes generating the first recognition result by performing object recognition based on the prescription data and the first image, and wherein the generating of the second recognition result includes generating the second recognition result by performing object recognition based on the prescription data and the second image.

The first recognition result may include at least one of location information, category information, width information, height information or a confidence value of a first object, wherein the calibrated second recognition result includes at least one of calibrated location information, calibrated width information, calibrated height information or a confidence value of a second object.

The first recognition result may include location information and category information of a first object, wherein the category information of the first object corresponds to one of medicines included in the prescription data.

The category information of the first object may correspond to one of different tablet shapes included in the medicine package.

The calibration parameter may define a positional relationship between at least part of pixels within an image captured by the first camera and at least part of pixels within an image captured by the second camera.

The method may further include, before capturing of the first image and the second image, receiving a fourth image captured by the first camera, receiving a fifth image captured by the second camera, and generating the calibration parameter based on the fourth image and the fifth image.

The generating of the third recognition result may include, in response to determination that a bounding box related to the first object and a bounding box related to the second object have a similarity equal to or greater than a predetermined first threshold value, removing one object with a lower confidence value between the first object and the second object, and adding the other object with a higher confidence value to the third recognition result.

The generating of the third recognition result may further include determining a similarity between the bounding box related to the first object and the bounding box related to the second object based on location information, width information, and height information of the first object, and calibrated location information, calibrated width information, and calibrated height information of the second object.

The generating of the third recognition result may include, in response to determination that category information of the first object is different from category information of the second object, a bounding box related to the first object and a bounding box related to the second object have a similarity less than a predetermined first threshold value, and an overlapping area between the bounding box related to the first object and the bounding box related to the second object is equal to or greater than a predetermined second threshold value, adding the first object and the second object to the third recognition result.

The medicine package may be one of a plurality of medicine packages dispensed based on the medicine information.

The medicine information may include prescription data or a medication count pattern.

The method may further include outputting a pass signal or a fail signal as a result of medicine package inspection.

According to an embodiment of the present disclosure, there is provided a computer program stored in a computer-readable recording medium for executing a method according to any one of claim 1 to claim 17 in a computer.

According to an embodiment of the present disclosure, there is provided a medicine package inspection device, including a first camera configured to capture a first image of a medicine package, a second camera configured to capture a second image of the medicine package, and a controller configured to obtain medicine information related to the medicine package, and inspect the medicine package based on the first image, the second image, and the medicine information.

According to some embodiments of the present disclosure, the present disclosure addresses the difficulty in achieving accurate object recognition/inspection with a single capturing device due to occlusion phenomena that may occur in the confined space within packaging. In addition, although imprinting on both sides of the tablet is an important factor for identifying the tablet, when the tablet is upside down, distinguishing the tablet may be difficult. By using a multi-angle imaging device according to the present disclosure, multi-angle images of the medicine package may be obtained to recognize the tablet. The number, shape, and type of tablets may be generated for each viewing angle. Subsequently, the information may be merged into a single result and output to indicate whether the result matches medicine information, thereby providing high inspection accuracy and significantly reducing the time and cost required during dispensing inspection.

According to some embodiments of the present disclosure, a plurality of tablets may be arranged within the narrow space of a medicine package, which potentially results in overlapping tablets during imaging. In such cases, object recognition for obscured tablets may be inaccurate. In addition, when an imprint is formed on only one side of the tablet, performing object recognition on the imprinted side of the tablet may achieve higher accuracy than performing object recognition on the unimprinted side of the tablet. Therefore, by using information of objects with a high confidence value based on the results of object recognition performed on a plurality of images, tablets occluded by other tablets and tablets with an imprint on only one side may be recognized with high accuracy.

According to some embodiments of the present disclosure, when three (3) or more tablets are in an overlapping state, the overlapping state of the tablets may be recognized/identified by comparing the result of object recognition performed based on a first image with the result of object recognition performed based on a second image. According to embodiments of the present disclosure, the overlapping relationship of the tablets may be clearly identified through object recognition using multi-view imaging.

The effects of the present disclosure are not limited to those described above, and other effects not mentioned will be clearly understood by those having ordinary knowledge in the art ('a person skilled in the art') from the description of the claims.

## BRIEF DESCRIPTION OF DRAWINGS

The embodiments of the present disclosure will be described with reference to the accompanying drawings described below, wherein similar reference numerals denote similar elements, but are not limited thereto.

FIG. 1 is a schematic view illustrating an example of a medicine package inspection device according to embodiments of the present disclosure;

FIG. 2 is a schematic view illustrating a configuration connected for communication between an information processing system, a medicine package inspection device, and a plurality of user terminals according to embodiments of the present disclosure;

FIG. 3 is a block diagram illustrating internal configuration of a user terminal and an information processing system according to embodiments of the present disclosure;

FIG. 4 is a view illustrating an example of capturing a medicine package by using a first camera and a second camera according to embodiments of the present disclosure;

FIG. 5 is a view illustrating an example of a process for performing object recognition according to embodiments of the present disclosure;

FIG. 6 is a view illustrating an example of performing object recognition by using prescription data according to embodiments of the present disclosure;

FIG. 7 is a view illustrating an example of comparing a calibrated second recognition result with a first recognition result;

FIG. 8 is a view illustrating an example of generating an inspection result based on images and medicine information according to embodiments of the present disclosure;

FIG. 9 is a view illustrating an example of a recognition result merging process when tablets are overlapping inside a medicine package;

FIG. 10 is a view illustrating an example of a recognition result merging process when category information is recognized differently for the same tablet;

FIG. 11 is a view illustrating an example of the recognition result merging process when three (3) different tablets are overlapping; and

FIG. 12 is a flowchart illustrating a medicine package inspection method according to embodiments of the present disclosure.

## DETAILED DESCRIPTION

Hereinafter, the specific details for implementing the present disclosure will be described in detail with reference to the accompanying drawings. However, in the following description, specific explanations of widely known functions or configurations will be omitted when the essence of the present disclosure is unnecessarily obscured.

In the accompanying drawings, identical or corresponding components are given the same reference numerals. In addition, in the description of the following embodiments, redundant descriptions of identical or corresponding components may be omitted. However, even though the description of components is omitted, such components are not intended to be excluded from any embodiment.

The advantages and features of the disclosed embodiments, and the methods for achieving the embodiments, will become apparent upon reference to the embodiments described below with reference to the accompanying drawings. However, the present disclosure is not limited to the embodiments disclosed below, but may be implemented in various different forms. The embodiments are provided merely to render the disclosure complete and to fully inform a person skilled in the art of the scope of the present disclosure.

The terms used in this specification shall be briefly explained, and the disclosed embodiments shall be described in detail. The terms used in the specification are selected from currently widely used general terminology in consideration of the functions within the present disclosure. However, the terms may vary depending on the intent of a person skilled in the art, case law, or the emergence of new technologies. Furthermore, in specific cases, terms are arbitrarily selected by the applicant. In such instances, the meaning thereof will be detailed in the corresponding description of the present disclosure. Therefore, the terms used in the present disclosure shall be defined based on the meaning thereof and the overall context of the present disclosure, not merely by designations of terms.

In this specification, singular expressions include plural expressions unless the context clearly specifies singular expressions. In addition, plural expressions include singular expressions unless the context clearly specifies plural expressions. Throughout the specification, when a part is described as including a component, it implies that additional components may also be included unless specifically stated to the contrary.

In addition, the terms 'module' or 'unit' used in the specification refer to software or hardware components, and the 'module' or 'unit' performs a specific role. However, 'module' or 'unit' is not limited to software or hardware. A 'module' or 'unit' may be configured to reside on an addressable storage medium or to execute one or more processors. Therefore, for example, 'module' or 'unit' include components such as software components, object-oriented software components, class components, and task components, processes, functions, attributes, procedures, subroutines, segments of program code, drivers, firmware, microcode, circuits, data, databases, data structures, tables, arrays, or variables. Functions provided within components and 'modules' or 'units' may be combined into a smaller number of components and 'modules' or 'units' or may be further separated into additional components and 'modules' or 'units'.

According to embodiments of the present disclosure, a 'module' or 'unit' may be implemented as a processor and a memory. The 'processor' shall be broadly interpreted to include a general-purpose processor, a central processing unit (CPU), a microprocessor, a digital signal processor (DSP), a controller, a microcontroller, a state machine, etc. In some environments, the 'processor' may also refer to an application-specific integrated circuit (ASIC), a programmable logic device (PLD), a field-programmable gate array (FPGA), etc. 'Processor' may also refer to combinations of processing devices, such as a combination of a DSP and a microprocessor, a combination of multiple microprocessors, a combination of one or more microprocessors coupled with a DSP core, or any other such combination. In addition, 'memory' shall be broadly interpreted to include any electronic component capable of storing electronic information. 'Memory' may also refer to various types of processor-readable media, such as random access memory (RAM), read-only memory (ROM), non-volatile random access memory (NVRAM), programmable read-only memory (PROM), erasable programmable read-only memory (EPROM), electrically erasable PROM (EEPROM), flash memory, magnetic or optical data storage devices, registers, and other types of processor-readable media. When the processor reads information from and/or records information in the memory, the memory is considered to be in an electronic communication state with the processor. A memory integrated into a processor is in an electronic communication state with the processor.

In addition, the terms such as first, second, A, B, (a), (b), etc., used in the following embodiments are used solely to distinguish one component from another and the nature, order, or sequence of the components, etc. are not limited by such terminology.

Furthermore, it will be understood that when a component is referred to as being "linked," "coupled," or "connected" to another component, the elements may be directly "coupled," "linked" or "connected" to each other, or another component may be "coupled," "linked" or "connected" between the components.

In this disclosure, 'each of a plurality of A' may refer to each of all components included in the plurality of A, or each of some components included in the plurality of A.

Also, it will be further understood that the terms "comprises," and/or "comprising," when used in the embodiments below, specify the presence of one or more components, steps, operations, and/or elements, but may not preclude the presence or addition of one or more other components, steps, operations, and/or elements.

In the present disclosure, 'tablet' may refer to a solid dosage form having a shape. For example, 'tablet' may be made from compressed powder or granules and may contain one or more active ingredients to maintain its dosage form. Tablets may be provided in various sizes and shapes. For example, a tablet may be circular, oval, square, cylindrical, or other shapes, and may be split into pieces from a solid dosage form. A tablet may be various colors depending on its materials and ingredients. Additionally, a tablet may have an imprint, such as printing, on one or both sides. The imprint may contain information such as logo, medicine name, dosage, or manufacturer, etc. The tablet surface may exhibit other surface markings such as scratches or cracks. The characteristics of tablets may be used to determine whether the tablets included in a medicine package match or differ from the prescribed tablets. For example, tablets may be implemented as dosage forms such as tablets, Extended-Release Tablets, Gastro-resistant Tablets, Triple Tablets, Oral Disintegrating Tablets, Sugar-coated Tablets, lozenges, vaginal tablets, enteric-coated tablets, capsules, soft gel capsules, etc., but are not limited thereto. Tablets may be packaged in blisters, pouches, sachets, bottles, etc.

According to disclosure, 'medicine package' may refer to a form of pharmaceutical packaging that protects the medication from damage, contamination, etc., and packages at least one tablet as a unit dose packaging for easy taking for patients based on a single dose. For example, the medicine package may be implemented as pouch, blister, sachet, container, syringe, etc., but it is not limited thereto. The surface of the medicine package may include important safety information for patient. For example, the surface of the medicine package may include information on the medication prescribed to the patient. Specifically, the information may include the type of medication in the package, the prescription date, dosage instructions, dosage, the patient's personal details, etc. Additionally, the surface of the medicine package may include information on the time of dosing. For example, when medication packaged in a plurality of medicine packages is to be taken in the morning, at noon, and in the evening, the surface of the medicine package may include information such as 'Morning' on the medicine package for medication needed in the morning, 'Noon' on the medicine package for medication needed at noon, and 'Evening' on the medicine package for medication needed in the evening. In addition, the surface of the medicine package may include information on the order of the medicine packages. For example, when a patient is prescribed twenty medicine packages, the surface of the medicine package may include information such as 'third medicine package out of twenty medicine packages' for the third medicine package.

In the present disclosure, 'medicine information' may refer to at least some of all data related to the medicine. For example, the medicine information may include various information related to the medicine such as the medicine product name, expiration date, ingredients, manufacturer, serial number, barcode, shape, form, color, imprint, etc. According to embodiments, the medicine information may be prescription data or a medication count pattern.

In the present disclosure, 'prescription data' may include information on medications prescribed to a patient. For example, prescription data may include patient information, the name of the prescribed medication, dosage, duration of use, dosage instructions, shape, form, color, imprint, etc. Additionally, prescription data may include information on which medication needs to be taken at a specific time. Additionally or alternatively, prescription data may include information on which medication included in a specific medicine package needs to be taken at a specific time.

According to the present disclosure, 'medication count pattern' may refer to information that consistently or characteristically arranges the number of tablets included within a plurality of medicine packages. For example, when the plurality of medicine packages include tablets in quantities such as 3-2-1-3-2-1-..., the medication count pattern may include information that every third medicine package contains three (3) tablets, followed by two (2) tablets, then one (1) tablet. In addition, the medication count pattern may be determined based on prescription data. In response to prescription data including information of arranging the number of tablets within a plurality of medicine packages, the medication count pattern may include arrangement information. Alternatively, the medication count pattern may be determined based on user-input information. Alternatively, the medication count pattern may be generated by a medicine package inspection device. Specifically, the medicine package inspection device may inspect the first n tablet packages out of a plurality of medicine packages to generate the medication count pattern.

In the present disclosure, 'category information' may refer to information that describes the type of medication. For example, category information may be an identifier that uniquely identifies a specific tablet. Alternatively, category information may be an identifier capable of identifying one of the shapes of different tablets included in the medicine package.

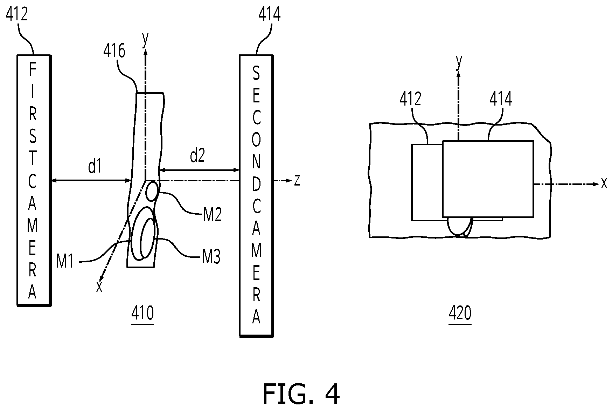

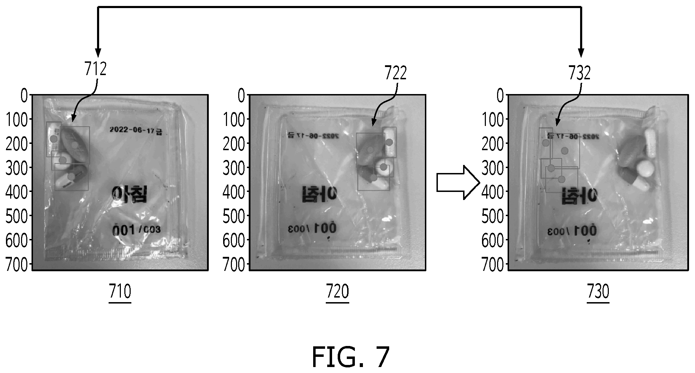

In the present disclosure, 'calibration parameter' may refer to a value related to a plurality of cameras and used to transform the object recognition results of images captured by the plurality of cameras. 'Calibration parameter' may define the positional relationship between some pixels within images captured by a plurality of different cameras. The method for generating calibration parameters will be described in more detail below with reference to FIG. 4 and FIG. 7.

In the present disclosure, 'bounding box' may refer to a rectangular area that surrounds an object detected in an image. A bounding box may be generated by an object recognition model. Specifically, tablets within a medicine package may be recognized as objects, and bounding boxes may be generated for the recognized tablets. In addition, bounding boxes may be generated for each recognized object, and a plurality of bounding boxes may be generated for a single object. Additionally, bounding boxes may be removed by comparison with other bounding boxes.

In the present disclosure, 'confidence value' may refer to information generated by an object recognition model that indicates the probability/degree of correctly recognizing an object in an image. For example, the confidence value may have a value between zero (0) and one (1). The closer the confidence value is to zero (0), the less accurately the object is recognized. The closer the confidence value is to one (1), the more accurately the object is recognized. In response to the object recognition model generating a plurality of bounding boxes for a single object, at least one of the plurality of bounding boxes may be selected based on the confidence value.

In the present disclosure, 'recognition result' may refer to information generated by the object recognition model, which may include location information, size information, width information, height information, information on the bounding box of the recognized object, etc. The recognition result may include information generated by the object recognition model based on the image and/or the image and prescription data. Additionally, the recognition result may include confidence value and/or category information.

Hereinafter, various embodiments of the present disclosure will be described in detail with reference to the accompanying drawings.

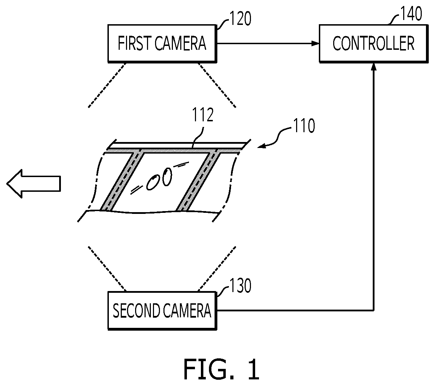

FIG. 1 is a schematic view illustrating an example of a medicine package inspection device according to embodiments of the present disclosure. As shown in the drawing, the medicine package inspection device may include a first camera 120, a second camera 130, and a controller 140. The medicine package inspection device may inspect a medicine package 112 based on the image of the medicine package 112 captured by the first camera 120 and the second camera 130 and medicine information. The first camera 120 and the second camera 130 may be disposed to capture different sides of the medicine package 112. Although FIG. 1 illustrates inspection of the medicine package by using two (2) cameras, the present disclosure is not limited thereto, but three (3) or more cameras may be used.

According to embodiments, a first image of the medicine package 112 captured by the first camera 120 and a second image of the medicine package 112 captured by the second camera 130 may be transmitted to the controller 140.

The controller 140 may inspect the medicine package 112 based on two (2) transmitted images and the medicine information related to the medicine package 112. The medicine information related to the medicine package 112 may include prescription data and/or medication count patterns.

According to embodiments, the medicine package inspection device may be attached to medicine package automatic packaging equipment. In this case, a plurality of medicine packages 110 packaged by the medicine package automatic packaging equipment may be supplied to the medicine package inspection device. As illustrated in the drawing, the plurality of medicine packages 110 may be inspected by moving in an arrow direction between the first camera 120 and the second camera 130. Specifically, after one medicine package 112, among the plurality of medicine packages 112, is captured by the first camera 120 and/or the second camera 130, a next medicine package among the plurality of packages 110 may move to an image capture point. The inspection result for each medicine package may be provided to a user in the form of a pass or fail determination. The inspection result for the medicine package may be provided in visual, auditory, tactile, or other forms.

According to embodiments, the controller 140 may generate a first recognition result by performing object recognition on the first image by using an object recognition model. Similarly, the controller 140 may generate a second recognition result by performing object recognition on the second image by using the object recognition model. The controller 140 may calibrate the second recognition result by using calibration parameters related to the first camera 120 and the second camera 130. The controller 140 may merge the first recognition result and the calibrated second recognition result to generate a third recognition result. The controller 140 may inspect the medicine package by comparing at least a part of the third recognition result with at least a part of the medicine information.

According to embodiments, the controller 140 may generate the first recognition result by performing object recognition on the first image by using the object recognition model. The controller 140 may generate the third image by transforming the second image by using calibration parameters related to the first camera 120 and the second camera 130. The controller 140 may generate the second recognition result by performing object recognition on the third image by using the object recognition model. The controller 140 may generate the third recognition result by merging the first recognition result and the second recognition result.

The controller 140 may inspect the medicine package 112 based on the third recognition result and the medicine information. For example, when at least part of the third recognition result and at least part of the medicine information fail to match, a failure signal may be output. For another example, when a comparison between a part of the third recognition results and a part of the medicine information reveals no mismatched information, a pass signal may be output. The detailed method for inspecting the medicine package 112 based on the third recognition result and the medicine information will be described below.

Such a configuration may solve the problem that accurate object recognition/inspection with a single capturing device is difficult due to overlapping/occlusion phenomena that may occur within the confined space of the packaging. The configuration may also solve the problem that, although the imprint on both sides of the tablet is a crucial element for identifying the tablet, distinguishing the tablet is difficult when the tablet is upside down. By using the multi-angle imaging device according to the present disclosure, multi-angle images of the medicine package may be obtained to recognize the tablets. The count, shape, and type of tablet may be generated for each angle to be merged into a single output to determine whether the output matches the medicine information, thereby providing high inspection accuracy by significantly reducing the time and cost required for dispensing inspection.

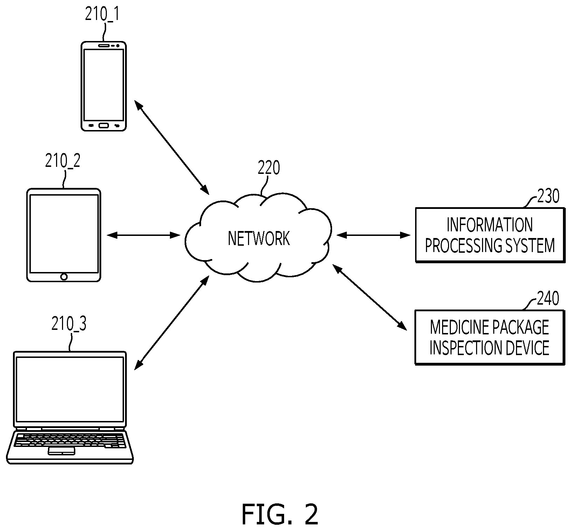

FIG. 2 is a schematic view illustrating a configuration connected for communication between an information processing system 230, a medicine package inspection device 240, and a plurality of user terminals 210_1, 210_2 and 210_3 according to embodiments of the present disclosure. As shown in the drawing, the plurality of user terminals 210_1, 210_2 and 210_3 may be connected to the information processing system 230 and the medicine package inspection device 240 capable of providing a medicine package inspection service via a network 220. The plurality of user terminals 210_1, 210_2 and 210_3 may include user terminals that receive the medicine package inspection service.

According to embodiments, the information processing system 230 may include one or more server devices and/or databases, which are capable of storing, providing, and executing computer-executable programs (e.g., downloadable applications) and data related to providing a medicine package inspection service, etc. or one or more distributed computing devices and/or distributed databases based on cloud computing services.

The medicine package inspection service provided by the information processing system 230 may be provided to a user through a medicine package inspection application, etc., installed on each of the plurality of user terminals 210_1, 210_2 and 210_3. For example, the information processing system 230 may provide or respond to information related to the medicine package inspection received from the user terminals 210_1, 210_2 and 210_3 and/or the medicine package inspection device 240 through the medicine package inspection application, etc.

According to embodiments, the information processing system 230 may inspect the medicine package based on the captured images and the medicine information related to the medicine package. The information processing system 230 may provide the result of the medicine package inspection to the user terminals 210_1, 210_2 and 210_3 and/or the medicine package inspection device 240.

The plurality of user terminals 210_1, 210_2 and 210_3 may communicate with the information processing system 230 and the medicine package inspection device 240 via the network 220. The network 220 may be configured to facilitate communication between the plurality of user terminals 210_1, 210_2 and 210_3, the information processing system 230, and the medicine package inspection device 240. Depending on installation environment, the network 220 may include wired networks such as Ethernet, Power Line Communication, telephone line communication devices, and RS-serial communication, wireless networks such as mobile communication networks, Wireless LAN (WLAN), Wi-Fi, Bluetooth, ZigBee, etc. or a combination thereof. The communication method is not limited thereto but may include not only communication methods using communication networks (e.g., mobile communication networks, wired internet, wireless internet, broadcast networks, satellite networks, etc.) that the network 220 may include, but also short-range wireless communication between the user terminals 210_1, 210_2 and 210_3.

Referring to FIG. 2, a mobile phone terminal 210_1, a tablet terminal 210_2, and a PC terminal 210_3 are shown as examples of the user terminals, but the present disclosure is not limited thereto. The user terminals 210_1, 210_2, and 210_3 may be any computing device capable of wired and/or wireless communication and capable of installing and executing an instant messaging application or a web browser, etc. For example, the user terminal may include an AI speaker, smartphone, mobile phone, navigation device, computer, laptop computer, digital broadcasting terminal, Personal Digital Assistant (PDA), Portable Multimedia Player (PMP), tablet PC, game console, wearable device, Internet of Things (IoT) device, virtual reality (VR) device, augmented reality (AR) device, set-top box, etc. In addition, FIG. 2 shows that three (3) user terminals 210_1, 210_2 and 210_3 communicate with the information processing system 230 and the medicine package inspection device 240 via the network 220. However, it is not limited thereto, but a different number of user terminals may also be configured to communicate with the information processing system 230 and the medicine package inspection device 240 via the network 220.

FIG. 2 illustrates that the medicine package inspection device 240 transmits medicine package images, etc., to the information processing system 230, and the information processing system 230 performs inspection of the medicine package, but it is not limited thereto. For example, the medicine package inspection device 240 may perform both image capturing and medicine package inspection as a stand-alone device without communicating with the information processing system 230, and directly provide the result of inspection to the plurality of user terminals 210_1, 210_2 and 210_3.

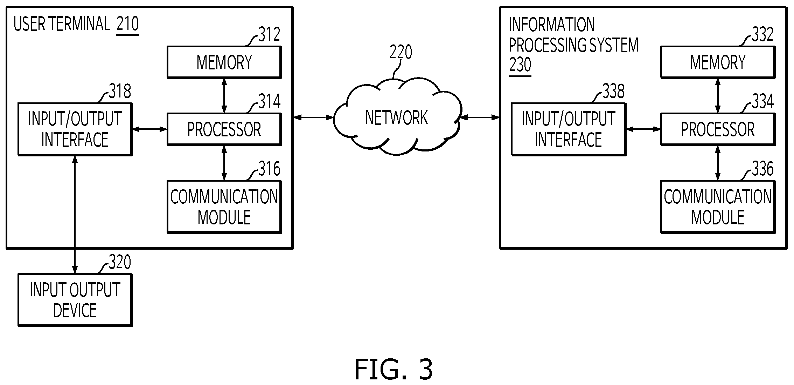

FIG. 3 is a block diagram illustrating the internal configuration of the user terminal and the information processing system according to embodiments of the present disclosure. The user terminal 210 may refer to any computing device capable of executing a medicine package inspection application, etc., and facilitating wired/wireless communication, and may include, for example, the mobile phone terminal 210_1, the tablet terminal 210_2, the PC terminal 210_3, etc., shown in FIG. 2. As shown in the drawing, the user terminal 210 may include a memory 312, a processor 314, a communication module 316, and an input/output interface 318. Similarly, the information processing system 230 may include a memory 332, a processor 334, a communication module 336, and an input/output interface 338. As shown in FIG. 3, the user terminal 210 and the information processing system 230 may be configured to communicate information and/or data via the network 220 by using respective communication modules 316 and 336. In addition, an input/output device 320 may be configured to input information and/or data into the user terminal 210 or output information and/or data generated by the user terminal 210 through the input/output interface 318.

The memories 312 and 332 may include non-transitory computer-readable storage media. According to embodiments, the memories 312 and 332 may include a permanent mass storage device such as a read-only memory (ROM), a disk drive, a solid state drive (SSD), a flash memory, etc. Alternatively, the permanent mass storage devices such as ROM, SSD, flash memory, disk drive, etc., may be a separate permanent storage device distinguished from a memory and included within the user terminal 210 or the information processing system 230. In addition, the memories 312 and 332 may store an operating system and at least one program code (e.g., code for a medicine package inspection application, etc. installed and operated in the user terminal 210).

Such software components may be loaded from a computer-readable storage medium separate from the memories 312 and 332. Such separate computer-readable storage media may include storage media directly connectable to the user terminals 210 and the information processing system 230 such as computer-readable storage media including floppy drives, disks, tapes, DVD/CD-ROM drives, memory cards, etc. As another example, software components may also be loaded into the memories 312 and 332 via a communication module rather than from a computer-readable storage medium. For example, at least one program may be loaded into the memories 312 and 332 based on computer programs installed by files provided via the network 220 by developers or file distribution systems that distribute application installation files.

The processors 314 and 334 may be configured to process instructions of the computer program by performing basic arithmetic, logic, and input/output operations. Instructions may be provided to the processors 314 and 334 by the memories 312 and 332 or the communication modules 316 and 336. For example, the processors 314 and 334 may be configured to execute instructions received according to program codes stored in a storage device such as the memories 312 and 332.

The communication modules 316 and 336 may provide a configuration or function for the user terminal 210 and the information processing system 230 to communicate with each other via the network 220, and may provide configuration or function for the user terminal 210 and/or the information processing system 230 to communicate with other user terminals or other systems (e.g., separate cloud system, etc.). For example, a request or data (e.g., medicine information related to a medicine package, a captured medicine package image, a recognition result, etc.) generated by the processor 314 of the user terminal 210 according to the program codes stored in a storage device such as the memory 312, etc., may be transmitted to the information processing system 230 via the network 220 under the control of the communication module 316. In reverse, control signals or instructions provided under the control of the processor 334 of the information processing system 230 may be received by the user terminal 210 via the communication module 316 of the user terminal 210 through the communication module 336 and the network 220. For example, the user terminal 210 may receive the medicine information related to the medicine package, captured images of the medicine package, recognition results, etc. from the information processing system 230.

The input/output interface 318 may be a means for interfacing with the input/output devices 320. For example, the input device may include devices such as a camera (including audio sensor and/or image sensor), a keyboard, a microphone, a mouse, etc., and the output device may include devices such as a display, a speaker, a haptic feedback device, etc. Alternatively, the input/output interface 318 may be a means for interfacing with a device that integrates input and output functions into a single configuration such as a touchscreen. For example, a service screen, etc. configured by using information and/or data provided by the information processing system 230 or another user terminal, as the processor 314 of the user terminal 210 processes instructions from the computer program loaded into memory 312, may be displayed on a display via the input/output interface 318. FIG. 3 illustrates that the input/output device 320 is not included in the user terminal 210. However, it is not limited thereto, but may be configured as a single integrated device with the user terminal 210. In addition, the input/output interface 338 of the information processing system 230 may be a means for interfacing with devices (not shown) connected to the information processing system 230 or devices for input or output included in the information processing system 230. In FIG. 3, the input/output interfaces 318 and 338 are shown as components separated from the processors 314 and 334, but the present disclosure is not limited thereto, but the input/output interfaces 318 and 338 may be configured to be included in the processors 314 and 334.

The user terminal 210 and the information processing system 230 may include more components than those shown in FIG. 3. However, it is not necessary to explicitly illustrate most conventional components. According to embodiments, the user terminal 210 may be implemented to include at least part of the input/output devices 320 described above. Additionally, the user terminal 210 may further include other components such as a transceiver, a Global Positioning System (GPS) module, a camera, various sensors, a database, etc. For example, when the user terminal 210 is a smartphone, the user terminal 210 may include components typically found in a smartphone, such as an accelerometer, a gyro sensor, an image sensor, a proximity sensor, a touch sensor, an ambient light sensor, a camera module, various physical buttons, buttons using a touch panel, input/output ports, a vibrator for vibration, and other various components.

During a time when a program for a medicine package inspection application, etc. is operated, the processor 314 may receive texts, images, video, audio, and/or motion, etc. received or selected through input devices such as touch screen, keyboard, audio sensor, and/or camera including image sensor, microphone, etc. connected to the input/output interface 318, may store the received texts, images, videos, audios, and/or motions, etc. in the memory 312, or provide the received texts, images, videos, audios, and/or motions, etc. to the information processing system 230 through the communication module 316 and the network 220.

The processor 314 of the user terminal 210 may be configured to manage, process, and/or store information and/or data received from the input/output device 320, other user terminals, the information processing system 230, and/or a plurality of external systems. Information and/or data processed by the processor 314 may be provided to the information processing system 230 via the communication module 316 and the network 220. The processor 314 of the user terminal 210 may transmit information and/or data to the input/output device 320 through the input/output interface 318 for output. For example, the processor 314 may display the received information and/or data on the screen of the user terminal 210.

The processor 334 of the information processing system 230 may be configured to manage, process, and/or store information and/or data received from a plurality of user terminals 210 and/or a plurality of external systems. The information and/or data processed by the processor 334 may be provided to the user terminal 210 through the communication module 336 and the network 220.

FIG. 4 is a view illustrating an example of capturing a medicine package 416 by using a first camera 412 and a second camera 414 according to embodiments of the present disclosure. As shown in the drawing, the first camera 412 may capture a first surface of the medicine package 416, and the second camera 414 may capture a second surface of the medicine package 416. The first surface may be the front surface of the medicine package 416, and the second surface may be the back surface of the medicine package 416. However, it is not limited thereto, but the first surface or the second surface may be one of a plurality of surfaces of the medicine package. The first image captured by the first camera 412 and the second image captured by the second camera 414 may be images of the same size (e.g., 720 x 720 px). Although two (2) cameras 412 and 414 are shown in FIG. 4, three (3) or more cameras may be used.

According to embodiments, referring to a perspective view 410 viewed from an x-axis direction showing that the first camera 412 and the second camera 414 capture the medicine package 416, the distance between the first camera 412 and the medicine package 416 may be a distance d1, and the distance between the second camera 414 and the medicine package 416 may be a distance d2. The distances d1 and d2 may vary depending on manufacturing tolerances, installation errors, temperature changes, and other factors at the time of manufacturing a medicine package inspection device. Therefore, the sizes of the medicine package 416 and the tablets M1, M2, and M3 of the image captured by the first camera 412 may differ from the sizes of the medicine package 416 and the tablets M1, M2, and M3 of the image captured by the second camera 414.

Referring to a side view 420 viewed from a z-axis direction showing that the first camera 412 and the second camera 414 capture the medicine package 416, the centers of the first camera 412 and the second camera 414 may be different from each other. In addition, the angle at which the first camera 412 views the medicine package 416 and the angle at which the second camera 414 views the medicine package 416 may differ from each other.

As described above, the location and the distance d1 of the first camera that captures the medicine package and the location and the distance d2 of the second camera that captures the medicine package may differ from each other. When the same tablet is captured by both the first camera 412 and the second camera 414, the size and location of the tablet in the first image captured by the first camera 412 may differ from the size and location of the tablet in the second image captured by the second camera 414. To merge the object recognition results from the first image and the second image, calibration parameters related to the first camera 412 and the second camera 414 may be used. The process of merging the object recognition results from the first image and the second image will be described below.

According to embodiments, the calibration parameters may be defined through a transformation matrix. For example, the transformation matrix may be expressed by Equation 1.

$$【\text{Equation 1}】$$

$$\begin{bmatrix} s_x & 0 & d_x \\ 0 & s_y & d_y \\ 0 & 0 & 1 \end{bmatrix}$$

$s_x$ and $s_y$ denote scale transformation parameters in the x-axis and y-axis directions, respectively, and $d_x$ and $d_y$ denote translation parameters in the x-axis and y-axis directions, respectively. The calibration parameters $s_x$, $s_y$, $d_x$ and $d_y$ may be generated and trained based on a plurality of images of at least one medicine package captured by the first camera 412 and the second camera 414. Specifically, object recognition may be performed by using an object recognition model on the plurality of images captured by the first camera 412 and the second camera 414. For each recognized object, the location information (e.g., center coordinate), width information, and height information within the image captured by the first camera 412 may be compared with the location information, width information, and height information within the image captured by the second camera 414.

According to embodiments, in consideration of $s_x$, $s_y$, $d_x$ and $d_y$ as trainable parameters, parameters may be optimized by using the multiple captured images and iterative learning. The calibration parameter generation/learning process may be described as assuming that object recognition is performed properly for the front-side capturing result (e.g., the first image) and the rear-side capturing result (e.g., the second image), and then mapping the bounding box of the object in the rear-side capturing result to the bounding box of the object in the front-side capturing result. Therefore, the calibration parameter generation/learning process may be considered the process of optimizing the elements of the transformation matrix by using a plurality of capturing results to ensure the mapping result is identical.

Calibration parameters may be generated or regenerated as needed. For example, calibration parameters may be generated immediately after the first and second cameras are installed. According to another example, calibration parameters may be generated each time the medicine package inspection device is powered on. According to yet another example, calibration parameters may be generated at predetermined periods (e.g., seasonally).

Mapping using calibration parameters will be described in detail with the following example. Calibration parameters are assumed to be generated as shown in Equation 2 through the calibration parameter generation/learning process.

【Equation 2】

$$M = \begin{bmatrix} -1 & 0 & 760 \\ 0 & 1 & 20 \\ 0 & 0 & 1 \end{bmatrix}$$

In the illustrated example, the medicine package 416 may include three (3) tablets M1 M2, and M3. Within the first image, the center coordinate of M1 may be [360,450], and the center coordinate of M2 may be [360,360]. In the second image, the center coordinate of M1 may be [400,430], and the center coordinate of M2 may be [397,340]. By using the calibration parameters, the center coordinate [400,430] of M1 and the center coordinate [397,340] of M2 within the second image may be transformed.

【Equation 3】

$$\begin{bmatrix} 360 \\ 450 \\ 1 \end{bmatrix} = \begin{bmatrix} -1 & 0 & 760 \\ 0 & 1 & 20 \\ 0 & 0 & 1 \end{bmatrix} \begin{bmatrix} 400 \\ 430 \\ 1 \end{bmatrix} \quad (약1)$$

$$\begin{bmatrix} 360 \\ 360 \\ 1 \end{bmatrix} = \begin{bmatrix} -1 & 0 & 760 \\ 0 & 1 & 20 \\ 0 & 0 & 1 \end{bmatrix} \begin{bmatrix} 397 \\ 340 \\ 1 \end{bmatrix} \quad (약2)$$

The center coordinates of M1 and M2 within the second image transformed by using the calibration parameter may be identical to the center coordinates of M1 ([360,450]) and M2 ([360,360]), respectively. By transforming the object recognition results by using the calibration parameters, the images captured from multiple angles may be merged to properly recognize objects occluded by overlapping phenomena.

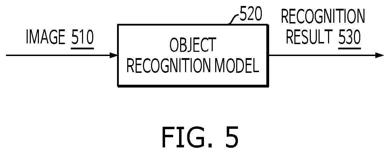

FIG. 5 is a view illustrating an example of the process for performing object recognition according to embodiments of the present disclosure. An image 510 may be a captured image of a medicine package including at least one tablet. For example, the image 510 may be one of the medicine package images captured by the first camera and the second camera of the medicine package inspection device. Alternatively, the image 510 may be an image calibrated by using calibration parameters from the medicine package image captured by the camera.

An object recognition model 520 may recognize at least one tablet included in the image 510 as an object without prescription data and generate a recognition result 530. For example, the object recognition model 520 may be Retinanet, YOLO, SSD, R-CNN, etc. The recognition result 530 may include information on each object recognized by the object recognition model 520. For example, the recognition result 530 may include location information and size information (width information, height information) of the bounding box, category information, confidence value, etc., of a specific object.

According to embodiments, the object recognition model 520 may learn various pharmaceutical data. Specifically, the object recognition model 520 may learn pharmaceutical data categorized by tablet type. For example, the pharmaceutical data may include the name, front image, back image, size information, etc., of each tablet. The object recognition model 520 may recognize the tablet object within the image 510 based on the trained data. In this case, the category information included in the recognition result 530 may correspond to specific pharmaceutical data (e.g., Tylenol 500 milligram (acetaminophen) Tablet).

According to another embodiment, the object recognition model 520 may not be able to identify which pharmaceutical product the tablet object is within the image 510, but may recognize and distinguish different tablet shapes included within the image 510. For example, when the image 510 includes a circular-shaped tablet, a triangular-shaped tablet, and an oval-shaped tablet, the category information included in the recognition result 530 may correspond to one of the different tablet shapes. In this case, the circular-shaped tablet object within the image 510 may be recognized as category information "class 1", the triangular-shaped tablet object may be recognized as category information "class 2", and the oval-shaped tablet object may be recognized as category information "class 3".

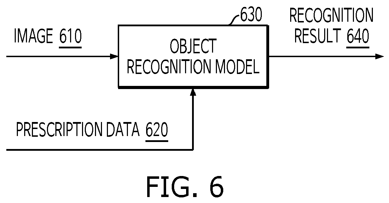

FIG. 6 is a view illustrating an example of performing object recognition by using prescription data 620 according to embodiments of the present disclosure. An object recognition model 630 may generate a recognition result 640 based on an image 610 and the prescription data 620. Specifically, the object recognition model 630 may recognize the location, shape, form, size, etc., of an object and assign one of the medicine types included in the prescription data 620 as category information to each recognized object. The object recognition model 630 may detect objects included in the image 610 and identify/recognize which type of medicine included in the prescription data 620 corresponds to the detected object.

The object recognition model 630 may be trained by using the prescription data 620 to recognize at least one tablet in the image 610 as an object. A recognition result 640 may include information on each object detected by the object recognition model 630. For example, the recognition result 640 may include location information and size information (width information, height information) on the bounding box, category information (e.g., No. 1 tablet included in the prescription data 620), confidence value, etc., of a specific object.

FIG. 7 is a view illustrating an example of comparison between a calibrated second recognition result 730 and a first recognition result 710. Referring to the first recognition result 710 obtained by performing object recognition by using an object recognition model on the first image according to embodiments, four (4) tablets within the first image may be recognized as a first set of objects 712. As shown in the drawing, the first image may be an image obtained by capturing the front of the medicine package. The recognized tablets may be displayed as bounding boxes on the first image, and a circle within each bounding box may indicate the center of the bounding box. The first recognition result 710 may include information on each of the first set of objects 712. For example, the first recognition result 710 may include location information (e.g., center coordinate), category information, size information (e.g., width information, and height information), confidence value, etc. for each object 712 in the first set.

In the similar manner, referring to a second recognition result 720 obtained by performing object recognition by using the object recognition model on the second image, four (4) tablets within the second image may be recognized as a second set of objects 722. As shown in the drawing, the second image may be an image obtained by capturing the back side of the same medicine package as the first image. The second recognition result 720 may include respective information on the second set of objects 722. For example, the second recognition result 720 may include location information (e.g., center coordinate), category information, size information (e.g., width information, and height information), confidence value, etc. for each of the second set of objects 722.

Although the first recognition result 710 and the second recognition result 720 are illustrated as objects detected on the captured image indicated by bonding boxes, but the present disclosure is not limited thereto. For example, the recognition result may include only information on each detected object without an image.

According to embodiments, the second recognition result 720 may be calibrated by using calibration parameters to generate a calibrated second recognition result 730. The calibration parameters may be used to calibrate/map the location information, width information, and height information of the second set of objects 722 included in the second recognition result 720 to correspond to the first image. The category information and the confidence information included in the second recognition result 730 may not be calibrated but may be included in the calibrated second recognition result 730. The calibrated second recognition result 730 may include information on the calibrated second set of objects 722. As illustrated in the drawing, the calibrated second set of objects 732 displayed on the second image may be moved to positions corresponding to the location of the tablet within the first image, rather than the location of the tablet within the second image.

The calibrated location information, calibrated width information, calibrated height information, category information, and confidence information on the calibrated second set of objects 732 included in the calibrated second recognition result 730 may be compared with the location information, width information, height information, category information, and confidence information on the first set of objects 712 included in the first recognition result 710. Through the comparison process, the calibrated second recognition result 730 and the first recognition result 710 may be merged to generate a third recognition result. The detailed process for merging the calibrated second recognition result 730 and the first recognition result 710 will be described below.

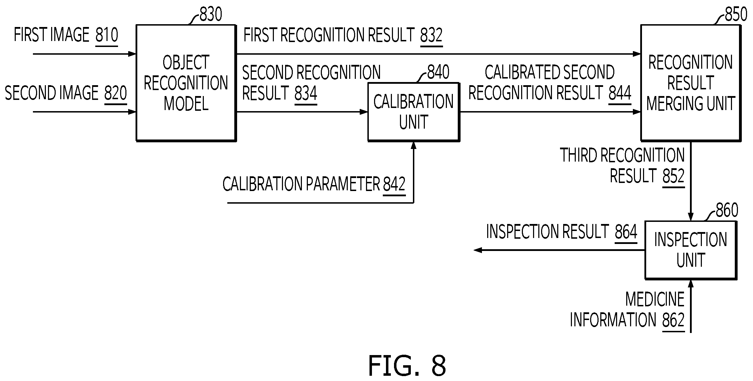

FIG. 8 is a view illustrating an example of generating an inspection result 864 based on images 810 and 820 and medicine information 862 according to embodiments of the present disclosure. A first recognition result 832 may be generated as the result of performing object recognition on a first image 810 by using an object recognition model 830. The first image 810 may be an image of the first surface of the medicine package captured by using the first camera. The first recognition result 832 may include location information, width information, size information, category information, confidence value, etc., for each object recognized by the object recognition model 830.

In the similar manner, a second recognition result 834 may be the result of object recognition performed on a second image 820 by using the object recognition model 830. The second image 820 may be an image of the second surface of the medicine package captured by using the second camera. The second recognition result 834 may include location information, width information, size information, category information, confidence value, etc., for objects recognized by the object recognition model 830.

According to embodiments, the object recognition model 830 may perform object recognition on each of the images 810 and 820 without additional information to generate recognition results 832 and 834. Alternatively, the object recognition model 830 may perform object recognition on each of the images 810 and 820 based on prescription data (not shown) to generate the recognition results 832 and 834. In this case, the recognition results 832 and 834 may include one of the medicine types included in the prescription data as category information.

According to embodiments, a calibrated second recognition result 844 may be generated by calibrating a second recognition result 834 by using a calibration parameter 842. Specifically, the calibrated second recognition result 844 may include information obtained by calibrating the location information, width information, and/or height information of the object included in the second recognition result 834 by using the calibration parameter 842. The category information and confidence information included in the second recognition result 834 may not be calibrated and may be included in the calibrated second recognition result 844 as it is. Through calibration using the calibration parameter 842, comparison between the first recognition result 832 and the second recognition result 834 may be available.

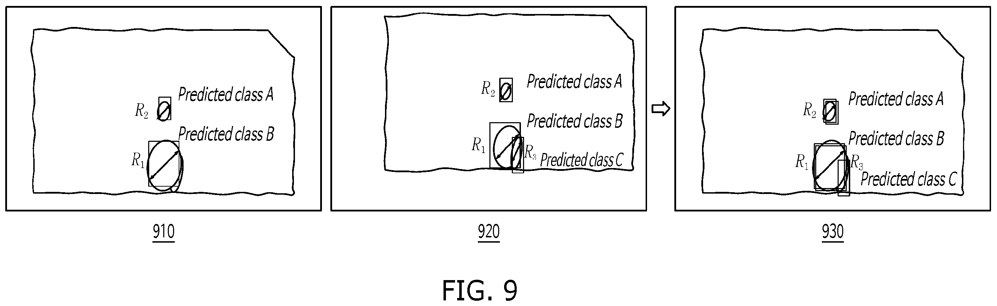

A recognition result merging unit 850 may merge the first recognition result 832 and the calibrated second recognition result 844 to generate a third recognition result 852. Specifically, the recognition result merging unit 850 may compare the bounding box of the object included in the calibrated second recognition result 844 with the bounding box of the object included in the first recognition result 832. The recognition result merging unit 850 may merge (e.g., union, intersection, etc.) at least one object included in the first recognition result 832 with at least one object included in the calibrated second recognition result 844. The third recognition result 852 may include location information, width information, height information, category information, confidence value, etc., on at least one object. Examples of the merging process will be described in detail in FIG. 9 to FIG. 11.

An inspection unit 860 may generate a medicine package inspection result 864 by comparing the third recognition result 852 with medicine information 862. The inspection result 864 for each medicine package may be provided to the user as a pass or fail determination. In the case of the failure determination, the specific reasons for failure (e.g., omission of some medicines, overpackaging of some medicines, packaging of other medicines, etc.) may be included in the inspection result 864. The medicine package inspection result 864 may be provided in visual, audible, tactile, or other forms.

According to embodiments, the medicine information 862 may be prescription data. Specifically, the prescription data may include the type of medication (i.e., category information) packaged in a single medicine package and the quantity information for each medication type. In this case, the category information on the objects within the third recognition result 852 may be compared with the category information (e.g., medicine type) included in the medicine information 862. As a result of the comparison, when the category information and the number of objects within the third recognition result 852 fail to match the category information (e.g., medicine type) and the number in the medicine information 862, the inspection unit 860 may issue a failure determination on the medicine package. When the category information and the number of objects within the third recognition result 852 match the category information (e.g., medicine type) and the number in the medicine information 862, the inspection unit 860 may issue a pass determination on the medicine package.

According to another embodiment, the medicine information 862 may include a predetermined medication count pattern. The medication count pattern may refer to information that consistently or characteristically arranges the number of tablets included in a plurality of medicine packages. The medication count pattern may be input by a user, determined based on prescription data, and/or generated by the medicine package inspection device. In this case, the inspection unit 860 may generate a medicine package inspection result 864 by comparing the number of objects with the medication count pattern, without using the category information included in the third recognition result 852. For example, when the medication count pattern is 3-2-1, the medicine package currently inspected by the inspection unit 860 corresponds to "2" sequence in the medication count pattern, and the number of objects included in the third recognition result 852 is two (2), the inspection unit 860 may issue a pass determination for inspection of the medicine package. When the number of objects included in the third recognition result 852 is not two (2), the inspection unit 860 may issue a failure determination on the medicine package.

According to embodiments, the inspection result 864 generated by the inspection unit 860 may be provided to the user. For example, the inspection result 864 may be displayed on a display mounted on the medicine package inspection device. Additionally or alternatively, the inspection result 864 may be displayed on a user terminal such as a mobile phone, a smartwatch, etc.

Although FIG. 8 illustrates the second recognition result 834 is calibrated by using the calibration parameter 842, but it is not limited thereto. For example, a third image (not shown) may be generated by transforming the second image 820 by using the calibration parameter 842. In this case, the information of each pixel within the image may be transformed by using the calibration parameter 842. Object recognition may be performed on the third image by using the object recognition model 830, thereby generating a second recognition result. The recognition result merging unit 850 may merge the first recognition result and the second recognition result to generate a third recognition result, and the inspection unit 860 may generate an inspection result based on the third recognition result and the medicine information 862.

FIG. 9 is a view illustrating an example of a recognition result merging process when tablets inside the medicine package overlap. As described above, by performing object recognition on the first image (e.g., the front image of the medicine package) captured by the first camera, a first recognition result 910 may be generated. Similarly, by performing object recognition on the second image (e.g., the back image of the medicine package) captured by the second camera, a second recognition result 920 may be generated. Subsequently, by calibrating the second recognition result 920 to generate a calibrated second recognition result, and merging the first recognition result 910 with the calibrated second recognition result, a third recognition result 930 may be generated.

As shown in the drawing, the first recognition result 910 may include information on a first set of objects ($R_1, R_2$).

For example, the first recognition result 910 may include information on the bounding box (e.g., location information, width information, and height information), category information (Class B), confidence value, etc. of $R_1$. Additionally, the first recognition result 910 may include information on the bounding box (e.g., location information, width information, and height information), category information (Class A), confidence value, etc. of $R_2$. An example of the first recognition result 910 is shown in Table 1 below.

[Table 1]

| Recognition Result Type | Tabl et | Location Informatio n | Category Information | Confidence Value |
|---|---|---|---|---|
| First Recognition Result | $R_1$ | [360,450] | B | 0.9 |
| First Recognition Result | $R_2$ | [360, 360] | A | 0.8 |

A second recognition result 920 may include information on a second set of objects ($R_1, R_2, R_3$). For example, the second recognition result 920 may include information on the bounding box (e.g., location information, width information, and height information), category information (Class B), confidence value, etc., $R_1$. Additionally, the second recognition result 920 may include information on the bounding box (e.g., location information, width information, and height information), category information (Class A), confidence value, etc., $R_2$. Additionally, the second recognition result 920 may include information on the bounding box (e.g., location information, width information, and height information), category information (Class C), confidence value, etc., $R_3$. An example of the second recognition result 920 is shown in Table 2 below.

[Table 2]

| Recognition Result Type | Tablet | Location Information | Category Information | Confidence Value |
|---|---|---|---|---|
| Second Recognition Result | $R_1$ | [400, 430] | B | 0.5 |
| Second Recognition Result | $R_2$ | [397, 340] | A | 0.8 |
| Second Recognition Result | $R_3$ | [410, 440] | C | 0.8 |

By calibrating the second recognition result 920 using the calibration parameter, a calibrated second recognition result (not shown) may be generated. For example, the calibration parameter may be $M = \begin{bmatrix} -1 & 0 & 760 \\ 0 & 1 & 20 \\ 0 & 0 & 1 \end{bmatrix}$. In this case, the information on the bounding box included in the second recognition result 920 may be calibrated, and the category information and the confidence value may not be calibrated. An example of the calibrated second recognition result is shown in Table 3 below.

[Table 3]

| Recognition Result Type | Tablet | Location Informatio n | Category Information | Confidence Value |
|---|---|---|---|---|
| Calibrated Second Recognition Result | $R_1$ | [360, 450] | B | 0.5 |
| Calibrated Second Recognition Result | $R_2$ | [363, 360] | A | 0.8 |
| Calibrated Second Recognition Result | $R_3$ | [350, 450] | C | 0.8 |

By comparing and merging the first recognition result 910 with the calibrated second recognition result, the third recognition result 930 may be generated. An example of comparing the first recognition result 910 with the calibrated second recognition result is shown in Table 4 below.

[Table 4]

| Recognition Result Type | Tablet | Location Information | Category Information | Confidence Value | Duplicate Determinatio n |
|---|---|---|---|---|---|
| First Recognition Result | $R_1$ | [360, 450] | B | 0.9 | O |
| Calibrated Second Recognition Result | $R_1$ | [360, 450] | B | 0.5 | O |

(continued)

| Recognition Result Type | Tablet | Location Information | Category Information | Confidence Value | Duplicate Determinatio n |
|---|---|---|---|---|---|
| First Recognition Result | $R_2$ | [360, 360] | A | 0.8 | O |
| Calibrated Second Recognition Result | $R_2$ | [363, 360] | A | 0.8 | O |
| First Recognition Result | $R_3$ | None | None | None | None |
| Calibrated Second Recognition Result | $R_3$ | [350, 450] | C | 0.8 | X |

According to embodiments, the recognition result comparison/merging process may include a step of determining the similarity between the first object included in the first recognition result 910 and the second object included in the calibrated second recognition result. Specifically, the recognition result merging process may include a step of determining the similarity based on the location information, width information, and height information of the bounding box of the object in the first recognition result 910, and the location information, width information, and height information of the calibrated bounding box of the object in the calibrated second recognition result.

According to embodiments, when the similarity between the bounding box of the first object included in the first recognition result 910 and the calibrated bounding box of the second object included in the calibrated second recognition result is equal to or greater than a predetermined first threshold value, the first object and the second object may be determined to be duplicate objects related to the same tablet (referred to as 'duplicate determination'). The first threshold value may be a value pre-input before the medicine package inspection device is installed and/or during the time of production of the medicine package inspection device. Alternatively, the first threshold value may be input before the medicine package inspection method is implemented. The input method of the first threshold value and the first threshold value are not limited thereto but may be determined/applied in various methods.

For the first object and the second object determined to be a duplicate, the confidence value of the first object may be compared with the confidence value of the second object. Information on the object with a higher confidence value may be added to the third recognition result 930, and information of the object with a lower confidence value may be removed or may not be added to the third recognition result 930. Referring to Table 4 for explanation, the similarity between the bounding box of $R_1$ included in the first recognition result 910 and the bounding box of $R_1$ included in the calibrated second recognition result may be the first threshold value or greater, which leads to a duplicate determination. The bounding box of $R_1$ included in the first recognition result 910 and the bounding box of $R_1$ included in the calibrated second recognition result may be determined to have similar locations and sizes. In this case, the confidence value of $R_1$ included in the first recognition result 910 may be 0.9, and the confidence value for $R_1$ included in the calibrated second recognition result may be 0.5. Therefore, the information on $R_1$ within the first recognition result 910 may be added to the third recognition result 930, and the information on $R_1$ within the calibrated second recognition result may be removed or may not be added to the third recognition result 930.

Additionally, the similarity between the bounding box of $R_2$ included in the first recognition result 910 and the bounding box of $R_2$ included in the calibrated second recognition result may be the first threshold value or greater, which leads to a duplicate determination. The confidence value of $R_2$ in the first recognition result 910 may be 0.8, and the confidence value of $R_2$ in the calibrated second recognition result may be 0.8. Therefore, one of the information on $R_2$ in the first recognition result 910 or the information on $R_2$ in the calibrated second recognition result may be added to the third recognition result 930, and the other may be removed or may not be added to the third recognition result 930.

According to embodiments, when category information of the first object included in the first recognition result 910 and category information of the second object included in the calibrated second recognition result are different, the similarity between the bounding box of the first object and the calibrated bounding box of the second object is below a predetermined first threshold value, and and the overlapping area between the bounding box of the first object and the bounding box of the second object is equal to or greater than a predetermined second threshold value, the first object and the second object may be determined to be related to different tablets in an occlusion state . In this case, both the first object information within the first recognition result 910 and the second object information within the calibrated second recognition result may be added to the third recognition result 930.

According to embodiments, the overlapping area between two (2) bounding boxes may be determined by using methods such as Intersection over Union (IoU), (Generalized-IoU (GIoU), Complete-IoU (CIoU), or Distance-IoU (DIoU), etc. The second threshold value may be a value pre-input before the medicine package inspection device is installed and/or during the time of production of the medicine package inspection device. Alternatively, the second threshold value may be input before the medicine package inspection method is implemented. The method of inputting the second threshold value is not limited thereto, but may be determined/applied in various ways.

Referring to Table 4 for explanation, $R_1$ included in the first recognition result 910 and $R_3$ included in the calibrated second recognition result may have different category information, B and C, respectively. The similarity between the bounding box of $R_1$ included in the first recognition result 910 and the bounding box of $R_3$ included in the calibrated second recognition result may be below a predetermined first threshold value (i.e., dissimilar in location and size), and the overlapping area between the bounding box of $R_1$ included in the first recognition result 910 and the bounding box of $R_3$ included in the calibrated second recognition result may be determined to be equal to or greater than a predetermined second threshold value. $R_3$ may be determined to be overlapped by $R_1$. In this case, both the information of $R_1$ included in the first recognition result 910 and the information of $R_3$ included in the calibrated second recognition result may be added to the third recognition result 930. An example of the third recognition result 930 is shown in Table 5 below.

[Table 5]

| Recognition Result Type | Tablet | Location Information | Category | Confidence Value |
|---|---|---|---|---|
| Third Recognition Result | $R_1$ | [360, 450] | B | 0.9 |
| Third Recognition Result | $R_2$ | [363, 360] | A | 0.8 |
| Third Recognition Result | $R_3$ | [350, 450] | C | 0.8 |

When tablets are in an overlapping state, tablets that may not be recognized as objects based on the first image may be recognized as objects based on the second image. For example, when $R_1$ and $R_3$ are overlapping, the first image may not recognize $R_3$ to be sufficient to identify $R_3$. However, the second image may recognize $R_3$ to identify the overlapping tablets. The invention according to embodiments of the present disclosure facilitates the identification of overlapping tablets through object recognition with multi-angle images.

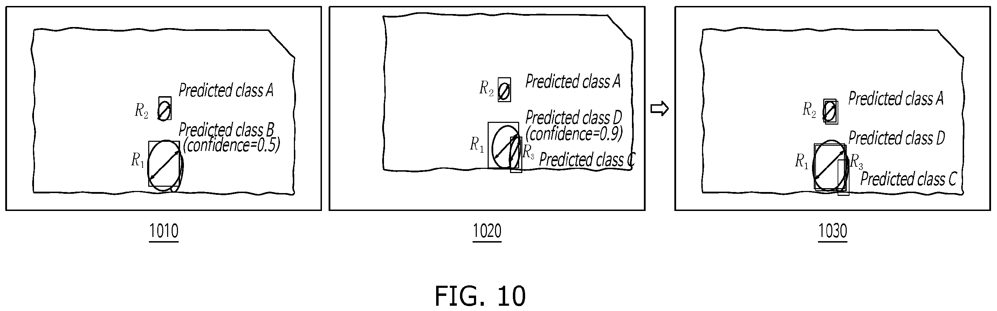

FIG. 10 is a view illustrating an example of the recognition result merging process when category information is recognized differently for the same tablet. As described above, performing object recognition on the first image captured by the first camera (e.g., the front image of the medicine package) may generate a first recognition result 1010. Similarly, by performing object recognition on the second image captured by the second camera (e.g., the back image of the medicine package), a second recognition result 1020 may be generated. Subsequently, by calibrating the second recognition result 1020 to generate a calibrated second recognition result, and merging the first recognition result 1010 with the calibrated second recognition result, a third recognition result 1030 may be generated.

The recognition results and the recognition merging result of $R_2$ and $R_3$ in the first recognition result 1010, the second recognition result 1020, and the third recognition result 1030 are identical to those of FIG. 9. Therefore, the detailed explanation thereof is omitted. The description of FIG. 10 will focus on $R_1$, which has a different recognition result from that in FIG. 9.

As shown in the drawing, the first recognition result 1010 may include information on $R_1$. For example, the first recognition result 1010 may include information (e.g., location information, width information, and height information) of the bounding box, category information (Class B), and a confidence value 0.5 of $R_1$. An example of the first recognition result 1010 is shown in Table 6 below.

[Table 6]

| Recognition Result Type | Tablet | Location Information | Category Information | Confidence Value |
|---|---|---|---|---|
| First Recognition Result | $R_1$ | [360, 450] | B | 0.5 |

The second recognition result 1020 may include information on $R_1$. For example, the second recognition result 1020 may include information on the bounding box (e.g., location information, width information, and height information), category information Class D, confidence value 0.9, etc., $R_1$. An example of the second recognition result 1020 is shown in Table 7 below.

[Table 7]

| Recognition Result Type | Tablet | Location Information | Category Information | Confidence Value |
|---|---|---|---|---|
| Second Recognition Result | $R_1$ | [400, 430] | D | 0.9 |

By calibrating the second recognition result 1020 using a calibration parameter, a calibrated second recognition result (not shown) may be generated. For example, the calibration parameter may be $M = \begin{bmatrix} -1 & 0 & 760 \\ 0 & 1 & 20 \\ 0 & 0 & 1 \end{bmatrix}$. In this

case, the information on the bounding box included in the second recognition result 1020 may be calibrated, and the category information and the confidence value may not be calibrated. An example of the calibrated second recognition result is shown in Table 8 below.

[Table 8]

| Recognition Result Type | Tablet | Location Information | Category Information | Confidence Value |
|---|---|---|---|---|
| Calibrated Second Recognition Result | $R_1$ | [360, 450] | B | 0.9 |

By comparing and merging the first recognition result 1010 with the calibrated second recognition result, a third recognition result 1030 may be generated. An example of comparing the first recognition result 1010 with the calibrated second recognition result is shown in Table 9 below.

[Table 9]

| Recognition Result Type | Tablet | Location Information | Category Information | Confidence Value | Duplicate Determination |
|---|---|---|---|---|---|
| First Recognition Result | $R_1$ | [360, 450] | B | 0.5 | O |
| Calibrated Second Recognition Result | $R_1$ | [360, 450] | D | 0.9 | O |

Referring to Table 9, the similarity between the bounding box of $R_1$ in the first recognition result 1010 and the bounding box of $R_1$ in the calibrated second recognition result may be the first threshold value or greater, which leads to a duplicate determination. The bounding boxes of $R_1$ in the first recognition result 1010 and $R_1$ in the calibrated second recognition result may be determined to have similar locations and sizes. In this case, the confidence value of $R_1$ in the first recognition result 1010 may be 0.5, and the confidence value of $R_1$ in the calibrated second recognition result may be 0.9. Therefore, information on $R_1$ included in the calibrated second recognition result may be added to the third recognition result 1030, and information on $R_1$ included in the first recognition result 1010 may be removed or may not be added to the third recognition result 1030.

The invention according to embodiments of the present disclosure may capture a plurality of images of a medicine package including at least one tablet, and based on the result of performing object recognition on the plurality of images, obtain information of objects with a high confidence value. In this case, the type of tablet may be identified more accurately than when a single image is captured. For example, when the category information on $R_1$ may be relatively inaccurately recognized as Class B in the first image, but the category information on $R_1$ may be relatively accurately recognized as Class D in the second image. In this case, by using the more accurate information, Class D, the type of tablet in the medicine package may be accurately identified.

In the case of the medicine package, a plurality of tablets may be arranged in a confined space and captured to be overlapping each other. In this case, object recognition for occluded tablets may be inaccurate. When an imprint is present on only one side of a tablet, performing object recognition on the imprinted side may achieve higher accuracy than performing object recognition on the side without an imprint. Therefore, by using information on objects with a high confidence value based on the results of object recognition performed on a plurality of images, tablets occluded by other tablets, and tablets with an imprint only on one side may be recognized with high accuracy.

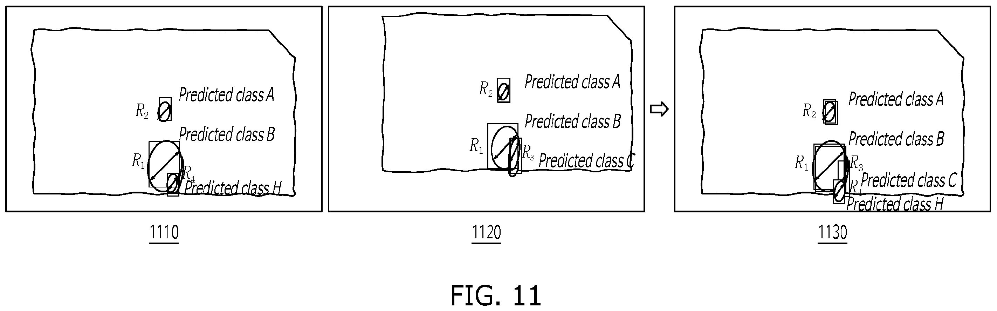

FIG. 11 is a view illustrating an example of the recognition result merging process when three (3) different tablets are overlapping. As described above, performing object recognition on the first image (e.g., the front image of the medicine package) captured by the first camera may generate a first recognition result 1110. In the similar manner, by performing object recognition on the second image (e.g., the back image of the medicine package) captured by the second camera, a second recognition result 1120 may be generated. Calibrating the second recognition result 1120 may generate a calibrated second recognition result, and merging the first recognition result 1110 with the calibrated second recognition result may generate a third recognition result 1130.

The recognition results and the merged recognition result of $R_1$, $R_2$, and $R_3$ in the first recognition result 1110, the second recognition result 1120, and the third recognition result 1130 may be identical to those in FIG. 9. Therefore, the detailed description thereof is omitted. The description of FIG. 11 will focus on $R_4$, which has a different recognition result from FIG. 9.

As shown in the drawing, the first recognition result 1110 may include information on $R_4$. For example, the first recognition result 1110 may include information on the bounding box (e.g., location information, width information, and height information), category information (Class H), confidence value, etc., $R_4$. The second recognition result 1120 may not include information on $R_4$. In the second recognition result 1120, $R_4$ may not be recognized as an object due to

occlusion by $R_1$ and $R_3$.

By calibrating the second recognition result 1120 using a calibration parameter, a calibrated second recognition result (not shown) may be generated. By comparing and merging the first recognition result 1110 and the calibrated second recognition result, the third recognition result 1130 may be generated. $R_4$ included in the first recognition result 1110, and all objects included in the calibrated second recognition result may have different category information and may be determined to have the similarity of the bounding boxes below a predetermined first threshold value (i.e., dissimilar in location and size). The overlapping area between the bounding box of $R_4$ included in the first recognition result 1110 and the bounding box of $R_1$ and/or $R_3$ included in the calibrated second recognition result may be determined to be equal to or greater than a predetermined second threshold value. $R_4$ may be determined to overlap $R_1$ and/or $R_3$. In this case, the information on $R_4$ in the first recognition result 1110 and the information on $R_1$ and/or $R_3$ in the calibrated second recognition result both may be added to the third recognition result 1130.

When three (3) or more tablets are in an overlapping state, the overlapping state of the tablets may be recognized/identified by comparing the result of object recognition performed based on the first image with the result of object recognition performed based on the second image. According to embodiments of the present disclosure, the overlapping relationship between the tablets may be clearly identified through object recognition through multi-angle imaging.

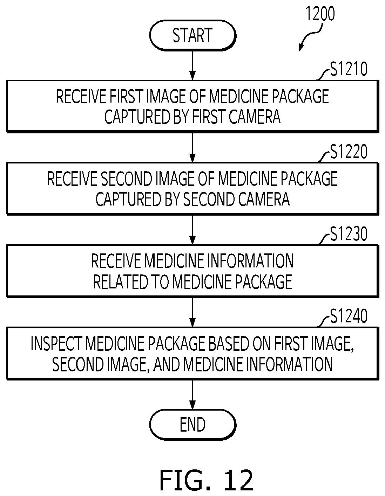

FIG. 12 is a flowchart illustrating a method 1200 for inspecting a medicine package according to embodiments of the present disclosure. The method 1200 may be performed by a controller (or at least one processor) of a medicine package inspection device and/or at least one processor of an information processing system. The method 1200 may initiate step S1210 where a processor receives a first image of the medicine package captured by a first camera. The first image may be an image obtained by capturing the first side of the medicine package. Additionally, the processor may receive a second image of the medicine package captured by a second camera in step S1220. The second image may be an image obtained by capturing the second side of the medicine package. The medicine package may include a plurality of tablets.

According to embodiments, the processor may receive medicine information related to the medicine package in step S1230. The medicine information may include prescription data or a medication count pattern. The medicine package may be one of a plurality of medicine packages dispensed based on the medicine information.

Thereafter, the processor may inspect the medicine package based on the first image, the second image, and the medicine information in step S1240.

According to embodiments, the step of inspecting the medicine package may include, performing object recognition on a first image by using an object recognition model to generate a first recognition result, performing object recognition on a second image by using the object recognition model to generate a second recognition result, and calibrating the second recognition result using calibration parameters related to the first camera and the second camera. The calibration parameters may define the positional relationship between at least some pixels within an image captured by the first camera and at least some pixels within an image captured by the second camera. Subsequently, the processor may inspect the medicine package based on the first recognition result, the calibrated second recognition result, and the medicine information.

According to embodiments, prior to capturing the first image and the second image, the processor may receive a fourth image captured by the first camera and a fifth image captured by the second camera. Subsequently, the processor may generate calibration parameters based on the fourth image and the fifth image.

According to embodiments, the step of generating a first recognition result may include performing object recognition based on the prescription data and the first image to generate the first recognition result. The first recognition result may include location information and category information of the first object. In addition, the category information of the first object may correspond to one of the medicine types included in the prescription data. Alternatively, the category information of the first object may correspond to one of different tablet shapes in the medicine package. Similarly, the step of generating a second recognition result may include performing object recognition based on the prescription data and the second image to generate the second recognition result.

According to embodiments, the first recognition result may include at least one of the location information, category information, width information, height information, or the confidence value of the first object. In addition, the calibrated second recognition result may include at least one of the calibrated location information, the category information, the calibrated width information, the calibrated height information, or the confidence value of the second object.

According to embodiments, the step of inspecting a medicine package based on the first recognition result, the calibrated second recognition result, and the medicine information may include a step of generating a third recognition result by merging the first recognition result and the calibrated second recognition result, and a step of inspecting the medicine package by comparing at least a part of the third recognition result with at least a part of the medicine information. The step of generating the third recognition result may include, in response to determination that the bounding box related to the first object and the bounding box related to the second object have the similarity equal to or greater than a predetermined first threshold value, a step of removing one with a lower confidence value between the first object and the second object and adding the other one with a higher confidence value to the third recognition result. The step of generating the third recognition result may include a step of determining the similarity between the bounding box related to the first object and the bounding box related to the second object based on the location information, the width information, and the height information of the first object, and the calibrated location information, the calibrated width information, and the calibrated height information of the second object.

Additionally or alternatively, the step of generating the third recognition result may include, in response to determination that the category information of the first object and the category information of the second object are different, and the similarity between the bounding box related to the first object and the bounding box related to the second object is below a predetermined first threshold value, and the overlapping area between the bounding box related to the first object and the bounding box related to the second object is determined to be a predetermined second threshold value or greater, adding the first object and the second object to the third recognition result.

According to another embodiment, the step of inspecting a medicine package may include, performing object recognition on a first image by using an object recognition model to generate a first recognition result, a step of transforming a second image into a third image by using calibration parameters related to a first camera and a second camera, a step of performing object recognition on the third image by using the object recognition model to generate a second recognition result, and a step of inspecting the medicine package based on the first recognition result, the second recognition result, and the medicine information.

According to embodiments, the processor may output a pass signal or a fail signal as the medicine package inspection result.

The flowchart and description provided above are merely examples, and some embodiments may be implemented differently. For example, in some embodiments, the order of each step may be changed, some steps may be performed repeatedly, some steps may be omitted, or some steps may be added.

The method described above may be provided as a computer program stored on a computer-readable recording medium for execution on a computer. The medium may continuously store a computer-executable program, or temporarily store the program for execution or download. Furthermore, the medium may be various recording or storage means in a form where one or more hardware components are combined, but may not be limited to media directly connected to a specific computer system and may also be distributed across a network. Examples of the media may include magnetic media such as hard disks, floppy disks, and magnetic tapes, optical recording media such as CD-ROMs and DVDs, magneto-optical media such as floptical disks, and other media configured to store program instructions and include ROM, RAM, flash memory, etc. Additionally, other examples of the media may include recording media or storage media managed by app stores that distribute applications, by sites that supply or distribute various software, servers, etc.

The methods, operations, or techniques of the present disclosure may be implemented by various means. For example, the techniques may be implemented in hardware, firmware, software, or a combination thereof. A person skilled in the art will understand that the various exemplary logic blocks, modules, circuits, and algorithmic steps described with respect to the present disclosure may be implemented by electronic hardware, computer software, or a combination thereof. To clearly illustrate this interchangeability of hardware and software, various exemplary components, blocks, modules, circuits, and steps are described above in general sense from the functional perspective. Whether such function is implemented as hardware or software may depend on design requirements imposed on the specific application and the overall system. A person skilled in the art may implement the function described in various ways for each specific application, but such implementations shall not be construed as departing from the scope of the present disclosure.

In hardware implementations, the processing units used to perform the techniques may include one or more ASICs, DSPs, digital signal processing devices (DSPDs), programmable logic devices (PLDs), field programmable gate arrays (FPGAs), processors, controllers, microcontrollers, microprocessors, electronic devices, other electronic units designed to perform the functions described in the present disclosure, computers, or combinations thereof.

Therefore, the various exemplary logic blocks, modules, and circuits described in connection with the present disclosure may be implemented or performed by any combination of a general-purpose processor, DSP, ASIC, FPGA or other programmable logic device, discrete gate or transistor logic, discrete hardware components, or those designed to perform the functions described in the present disclosure. The general-purpose processor may be a microprocessor, but alternatively, the processor may be any conventional processor, controller, microcontroller, or state machine. The processor may also be implemented as a combination of computing devices, such as a DSP and a microprocessor, a plurality of microprocessors, one or more microprocessors coupled with a DSP core, or any other combination of components.

In firmware and/or software implementation, techniques may be implemented as instructions stored in random access memory (RAM), read-only memory (ROM), non-volatile random access memory (non-volatile RAM (NVRAM)), programmable read-only memory (PROM), erasable programmable read-only memory (EPROM), electrically erasable PROM (EEPROM), flash memory, and compact disc (CD), computer-readable medium such as magnetic or optical data storage devices. The instructions may be executable by one or more processors, or may allow the processor(s) to perform specific aspects of the functions described in the present disclosure.

When implemented as software, the techniques described above may be stored on a computer-readable medium or transmitted via a computer-readable medium as one or more instructions or codes. Computer-readable media may include both computer storage media and communication media with any medium that facilitates the transmission of a computer program from one location to another. Storage media may also be any available medium accessible by a computer. As a non-limiting example, such computer-readable media may be used to transmit and store RAM, ROM, EEPROM, CD-ROM or other optical disk storage, magnetic disk storage or other magnetic storage devices, or any desired program code in the form of instructions or data structures and may include any other medium accessible by a computer. In addition, any medium accessed by a computer may appropriately be termed a computer-readable medium.

For example, when software is transmitted from a website, server, or other remote source using coaxial cable, fiber optic cable, twisted pair cable, digital subscriber line (DSL), or wireless technologies such as infrared, radio, and microwave, the coaxial cable, the fiber optic cable, the twisted pair cable, the digital subscriber line, or the wireless technologies such as infrared, radio, and microwave may be included within the definition of the medium. The terms 'disk' and 'disc' used in the present disclosure may include CD, laser disc, optical disc, digital versatile disc (DVD), floppy disk, and Blu-ray disc, and the disks may typically reproduce data magnetically. However, the discs may reproduce data optically using a laser. The above combinations shall be included within the scope of computer-readable media.

The software module may reside in RAM memory, flash memory, ROM memory, EPROM memory, EEPROM memory, registers, hard disk, removable disk, CD-ROM, or any other known form of storage medium. An exemplary storage medium may be connected to a processor to allow the processor to read information from the storage medium or record information to the storage medium. Alternatively, the storage medium may be integrated into the processor. The processor and the storage medium may reside within an ASIC. The ASIC may reside within a user terminal. Alternatively, the processor and the storage medium may exist as separate components in a user terminal.

Although the embodiments described above have been described as using aspects of the subject matter disclosed in one or more standalone computer systems, the present disclosure is not limited thereto and may also be implemented in connection with any computing environment, such as a network or a distributed computing environment. In addition, the aspects of the subject matter in the present disclosure may be implemented in a plurality of processing chips or devices, and the storage may similarly be affected across the plurality of devices. The devices may include PCs, network servers, and portable devices.

Although the present disclosure has been described with reference to some embodiments, various modifications and changes may be made without departing from the scope of the present disclosure as understood by a person skilled in the art to which the present disclosure pertains. Furthermore, such modifications and changes shall be considered to fall within the scope of the appended claims.

**Claims**

1.   A method for inspecting a medicine package performed by at least one processor, the method comprising:

   receiving a first image of a medicine package captured by a first camera;
   receiving a second image of the medicine package captured by a second camera;
   receiving medicine information related to the medicine package; and
   inspecting the medicine package based on the first image, the second image, and the medicine information, wherein the medicine package includes a plurality of tablets.

2.   The method as claimed in claim 1, wherein the inspecting the medicine package comprises:

   performing object recognition on the first image by using an object recognition model and generating a first recognition result;
   performing object recognition on the second image by using the object recognition model and generating a second recognition result;
   calibrating the second recognition result by using a calibration parameter related to the first camera and the second camera; and
   inspecting the medicine package based on the first recognition result, the calibrated second recognition result, and the medicine information.

3.   The method as claimed in claim 1, wherein the inspecting the medicine package comprises:

   performing object recognition on the first image by using an object recognition model and generating a first recognition result;
transforming the second image into a third image by using a calibration parameter related to the first camera and the second camera;
performing object recognition on the third image by using the object recognition model and generating a second recognition result; and
inspecting the medicine package based on the first recognition result, the second recognition result, and the medicine information.

4. The method as claimed in claim 1, wherein the first image is obtained by capturing a first side of the medicine package, and
wherein the second image is obtained by capturing a second side of the medicine package.

5. The method as claimed in claim 2, wherein the inspecting the medicine package based on the first recognition result, the calibrated second recognition result, and the medicine information comprises:

generating a third recognition result by merging the first recognition result and the calibrated second recognition result; and
inspecting the medicine package by comparing at least part of the third recognition result with at least part of the medicine information.

6. The method as claimed in claim 2, wherein the medicine information comprises prescription data,
wherein the generating the first recognition result comprises:

generating the first recognition result by performing object recognition based on the prescription data and the first image, and
wherein the generating the second recognition result comprises:
generating the second recognition result by performing object recognition based on the prescription data and the second image.

7. The method as claimed in claim 5, wherein the first recognition result includes at least one of location information, category information, width information, height information or a confidence value of a first object, and
wherein the calibrated second recognition result includes at least one of calibrated location information, calibrated width information, calibrated height information or a confidence value of a second object.

8. The method as claimed in claim 6, wherein the first recognition result includes location information and category information of a first object, and
wherein the category information of the first object corresponds to one of medicines included in the prescription data.

9. The method as claimed in claim 7, wherein the category information of the first object corresponds to one of different tablet shapes included in the medicine package.

10. The method as claimed in claim 2, wherein the calibration parameter defines a positional relationship between at least part of pixels within an image captured by the first camera and at least part of pixels within an image captured by the second camera.

11. The method as claimed in claim 10, further comprising, before capturing the first image and the second image,

receiving a fourth image captured by the first camera;
receiving a fifth image captured by the second camera; and
generating the calibration parameter based on the fourth image and the fifth image.

12. The method as claimed in claim 7, wherein the generating the third recognition result comprises:
in response to determination that a bounding box related to the first object and a bounding box related to the second object have a similarity equal to or greater than a predetermined first threshold value, removing one object with a lower confidence value between the first object and the second object, and adding the other object with a higher confidence value to the third recognition result.

13. The method as claimed in claim 12, wherein the generating the third recognition result further comprises:

determining a similarity between the bounding box related to the first object and the bounding box related to the second object based on location information, width information, and height information of the first object, and calibrated location information, calibrated width information, and calibrated height information of the second object.

14. The method as claimed in claim 7, wherein the generating the third recognition result comprises:
in response to determination that category information of the first object is different from category information of the second object, a bounding box related to the first object and a bounding box related to the second object have a similarity less than a predetermined first threshold value, and an overlapping area between the bounding box related to the first object and the bounding box related to the second object is equal to or greater than a predetermined second threshold value, adding the first object and the second object to the third recognition result.

15. The method as claimed in claim 1, wherein the medicine package is one of a plurality of medicine packages dispensed based on the medicine information.

16. The method as claimed in claim 1, wherein the medicine information includes prescription data or a medication count pattern.

17. The method as claimed in claim 1, further comprising outputting a pass signal or a fail signal as a result of medicine package inspection.

18. A non-transitory computer-readable recording medium storing instructions for executing the method of claim 1 on a computer.

19. A medicine package inspection device, comprising:

a first camera configured to capture a first image of a medicine package;
a second camera configured to capture a second image of the medicine package; and
a controller configured to obtain medicine information related to the medicine package, and inspect the medicine package based on the first image, the second image, and the medicine information.

FIG. 1

210_1

210_2

220

230

INFORMATION
PROCESSING SYSTEM

NETWORK

210_3

240

MEDICINE PACKAGE
INSPECTION DEVICE

FIG. 2

USER TERMINAL 210

MEMORY ⟋312

INPUT/OUTPUT INTERFACE ⟋318

PROCESSOR ⟋314

COMMUNICATION MODULE ⟋316

INPUT OUTPUT DEVICE ⟋320

NETWORK 220

INFORMATION PROCESSING SYSTEM 230

MEMORY ⟋332

INPUT/OUTPUT INTERFACE ⟋338

PROCESSOR ⟋334

COMMUNICATION MODULE ⟋336

EP 4 756 822 A1

FIG. 3

FIG. 4

IMAGE 510 → | OBJECT RECOGNITION MODEL ⌐520 | → RECOGNITION RESULT 530

FIG. 5

IMAGE <u>610</u> → OBJECT RECOGNITION MODEL ⟋630 → RECOGNITION RESULT <u>640</u>

PRESCRIPTION DATA <u>620</u>

## FIG. 6

FIG. 7

FIRST IMAGE 810 → OBJECT RECOGNITION MODEL 830 → FIRST RECOGNITION RESULT 832

SECOND IMAGE 820 →

SECOND RECOGNITION RESULT 834 → CALIBRATION UNIT 840 → CALIBRATED SECOND RECOGNITION RESULT 844 → RECOGNITION RESULT MERGING UNIT 850

CALIBRATION PARAMETER 842

THIRD RECOGNITION RESULT 852

INSPECTION RESULT 864 ← INSPECTION UNIT 860

MEDICINE INFORMATION 862

FIG. 8

FIG. 9

FIG. 10

1110      1120      1130

FIG. 11

1200

START

RECEIVE FIRST IMAGE OF MEDICINE PACKAGE
CAPTURED BY FIRST CAMERA ⌐S1210

RECEIVE SECOND IMAGE OF MEDICINE PACKAGE
CAPTURED BY SECOND CAMERA ⌐S1220

RECEIVE MEDICINE INFORMATION
RELATED TO MEDICINE PACKAGE ⌐S1230

INSPECT MEDICINE PACKAGE BASED ON FIRST IMAGE,
SECOND IMAGE, AND MEDICINE INFORMATION ⌐S1240

END

FIG. 12

## INTERNATIONAL SEARCH REPORT

| International application No. |
| --- |
| **PCT/KR2023/020202** |

**A. CLASSIFICATION OF SUBJECT MATTER**

G16H 20/13(2018.01)i; G06V 10/10(2022.01)i; G06V 30/41(2022.01)i; G06V 10/75(2022.01)i; G06V 10/74(2022.01)i

According to International Patent Classification (IPC) or to both national classification and IPC

**B. FIELDS SEARCHED**

Minimum documentation searched (classification system followed by classification symbols)

G16H 20/13(2018.01); B07C 5/34(2006.01); G01N 21/95(2006.01); G06Q 10/10(2012.01); G06V 10/10(2022.01); G16C 10/00(2019.01); H04N 13/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 약포(charta), 알약(pill), 검수(checking), 카메라(camera), 이미지(image)

**C. DOCUMENTS CONSIDERED TO BE RELEVANT**

| Category* | Citation of document, with indication, where appropriate, of the relevant passages | Relevant to claim No. |
| --- | --- | --- |
| X | KR 10-2023-0042917 A (J&J TECH INC.) 30 March 2023 (2023-03-30) See paragraphs [0031], [0047]-[0059] and [0073]-[0110]. | 1,4,15-19 |
| Y | | 2-3,6,8,10 |
| A | | 5,7,9,11-14 |
| Y | KR 10-2016-0138079 A (YUYAMA MFG. CO., LTD.) 02 December 2016 (2016-12-02) See paragraphs [0207], [0211] and [0217]. | 2-3,6,8,10 |
| Y | KR 10-2016-0060403 A (SAMSUNG ELECTRONICS CO., LTD.) 30 May 2016 (2016-05-30) See paragraph [0030]. | 10 |
| A | KR 10-2022-0067370 A (MEDILITY INC.) 24 May 2022 (2022-05-24) See paragraphs [0029]-[0054]; and figures 1-3. | 1-19 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **07 November 2024** | **07 November 2024** |

| Name and mailing address of the ISA/KR | Authorized officer |
| --- | --- |
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/020202**

| C. | DOCUMENTS CONSIDERED TO BE RELEVANT | |
|---|---|---|
| Category* | Citation of document, with indication, where appropriate, of the relevant passages | Relevant to claim No. |
| A | KR 10-2022-0167613 A (JVM CO., LTD.) 21 December 2022 (2022-12-21) <br> See paragraphs [0031]-[0054]; and figures 1-4. | 1-19 |

Form PCT/ISA/210 (second sheet) (July 2022)

# EP 4 756 822 A1

| | INTERNATIONAL SEARCH REPORT | International application No. |
|---|---|---|
| | Information on patent family members | PCT/KR2023/020202 |

| Patent document cited in search report | | | Publication date (day/month/year) | Patent family member(s) | | | Publication date (day/month/year) |
|---|---|---|---|---|---|---|---|
| KR | 10-2023-0042917 | A | 30 March 2023 | None | | | |
| KR | 10-2016-0138079 | A | 02 December 2016 | AU | 2015-239178 | A1 | 08 October 2015 |
| | | | | AU | 2015-239178 | B2 | 05 December 2019 |
| | | | | CN | 106415251 | A | 15 February 2017 |
| | | | | CN | 106415251 | B | 19 May 2020 |
| | | | | JP | 2019-076787 | A | 23 May 2019 |
| | | | | JP | 2020-011122 | A | 23 January 2020 |
| | | | | JP | 2020-157077 | A | 01 October 2020 |
| | | | | JP | 2022-010060 | A | 14 January 2022 |
| | | | | JP | 6607185 | B2 | 20 November 2019 |
| | | | | JP | 6753479 | B2 | 09 September 2020 |
| | | | | JP | 6939865 | B2 | 22 September 2021 |
| | | | | JP | 7238942 | B2 | 14 March 2023 |
| | | | | KR | 10-2276711 | B1 | 13 July 2021 |
| | | | | US | 10969218 | B2 | 06 April 2021 |
| | | | | US | 2017-0264867 | A1 | 14 September 2017 |
| | | | | WO | 2015-152225 | A1 | 08 October 2015 |
| KR | 10-2016-0060403 | A | 30 May 2016 | CN | 105635719 | A | 01 June 2016 |
| | | | | CN | 105635719 | B | 13 August 2019 |
| | | | | EP | 3024229 | A2 | 25 May 2016 |
| | | | | EP | 3024229 | A3 | 08 June 2016 |
| | | | | EP | 3024229 | B1 | 16 September 2020 |
| | | | | JP | 2016-100899 | A | 30 May 2016 |
| | | | | JP | 2021-005890 | A | 14 January 2021 |
| | | | | JP | 6767743 | B2 | 14 October 2020 |
| | | | | JP | 7163345 | B2 | 31 October 2022 |
| | | | | KR | 10-2281184 | B1 | 23 July 2021 |
| | | | | US | 10506213 | B2 | 10 December 2019 |
| | | | | US | 11140374 | B2 | 05 October 2021 |
| | | | | US | 2016-0150211 | A1 | 26 May 2016 |
| | | | | US | 2020-0053338 | A1 | 13 February 2020 |
| KR | 10-2022-0067370 | A | 24 May 2022 | CA | 3198777 | A1 | 27 May 2022 |
| | | | | CN | 116457840 | A | 18 July 2023 |
| | | | | JP | 2023-549252 | A | 22 November 2023 |
| | | | | JP | 7474546 | B2 | 25 April 2024 |
| | | | | KR | 10-2022-0067363 | A | 24 May 2022 |
| | | | | KR | 10-2505705 | B1 | 03 March 2023 |
| | | | | KR | 10-2607174 | B1 | 28 November 2023 |
| | | | | US | 2023-0306630 | A1 | 28 September 2023 |
| | | | | WO | 2022-108044 | A1 | 27 May 2022 |
| KR | 10-2022-0167613 | A | 21 December 2022 | WO | 2022-265320 | A1 | 22 December 2022 |

Form PCT/ISA/210 (patent family annex) (July 2022)